# EUROPÄISCHE PATENTSCHRIFT

(11) **EP 3 716 921 B1**
(45) Veröffentlichungstag und Bekanntmachung des Hinweises auf die Patenterteilung: **30.06.2021**
(21) Anmeldenummer: 18812147.9
(22) Anmeldetag: 30.11.2018
(51) Int. Cl.: A61F 5/01

(54) **VERSTELLEINRICHTUNG UND ORTHESE MIT EINER VERSTELLEINRICHTUNG**
ADJUSTMENT DEVICE AND ORTHOSIS WITH AN ADJUSTMENT DEVICE
DISPOSITIF D'AJUSTEMENT ET ORTHESE COMPRENANT UN DISPOSITIF D'AJUSTEMENT

(30) Priorität: 01.12.2017 DE 102017128613
(43) Veröffentlichungstag der Anmeldung: 07.10.2020
(73) Patentinhaber: Ottobock SE & Co. KGaA, 37115 Duderstadt (DE)
(72) Erfinder: SCHILLING, Matthias, 37345 Weißenborn-Lüderode (DE); MÜLLER, André, 37115 Duderstadt (DE)
(74) Vertreter: Gramm, Lins & Partner Patent- und Rechtsanwälte PartGmbB
(86) Internationale Anmeldenummer: PCT/EP2018/083163
(87) Internationale Veröffentlichungsnummer: WO 2019/106153

(56) Entgegenhaltungen:
- EP-A1- 1 588 678
- WO-A1-90/00879
- US-A- 5 848 979
- US-A1- 2007 066 923

## Beschreibung

Die Erfindung betrifft eine Verstelleinrichtung zur verstellbaren Festlegung zumindest zweier orthetischer Komponenten zueinander, mit einem ersten Halter, der eine Befestigungseinrichtung zur Festlegung einer ersten orthetischen Komponente aufweist, und einem zweiten Halter, der eine zweite Befestigungseinrichtung zur Befestigung einer zweiten orthetischen Komponente aufweist, wobei die Halter in zumindest zwei Orientierungen zueinander verschieblich und miteinander fixierbar gelagert sind sowie eine Orthese mit einer solchen Verstelleinrichtung.

Orthesen sind Hilfseinrichtungen, die an dem Körper eines Patienten getragen werden. Sie dienen zur Stabilisierung von Gelenken, der Entlastung einer Gliedmaße, zur Einschränkung einer Relativbewegung zweier Gliedmaßen zueinander, beispielsweise durch Begrenzung einer Extension und Flexion oder auch zur Führung einer Bewegung. Ebenfalls existieren Orthesen, die eine Korrekturfunktion ausführen, beispielsweise um bei arthrotischen Beschwerden Gelenkkomponenten zueinander auszurichten oder einzelne Gelenkbereiche zu entlasten. Orthesen können gelenkübergreifend an einer Gliedmaße befestigt werden oder aber auch an einem Rumpf eines Nutzers angelegt werden. Häufig weisen Orthesen mehrere Komponenten auf, die zueinander ausgerichtet werden müssen, ebenso ist es notwendig, dass die Orthese an den Nutzer angepasst wird, sodass die einzelnen Komponenten zueinander orientiert und ausgerichtet werden müssen, damit die jeweilige Orthese ihre vorgegebene oder gewünschte Funktion bestmöglich erfüllen kann.

Zur Fertigung von Orthesen werden häufig Standardkomponenten eingesetzt, die für die jeweilige Funktion und an den jeweiligen Nutzer angepasst werden. Ein wichtiger Gesichtspunkt bei der Gewährleistung einer optimalen Funktionsfähigkeit einer Orthese ist die korrekte Ausrichtung der Komponenten zueinander und der richtige Sitz der Orthese an dem jeweiligen Nutzer, um einerseits die gewünschten Funktionen bereitzustellen und andererseits den Tragekomfort nicht einzuschränken.

Zur Anpassung beispielsweise einer Fußhebeorthese ist es vorgesehen, dass eine Unterschenkelschiene an die Form des Unterschenkels angepasst wird, indem die Unterschenkelschiene plastisch geformt wird. Eine Fußplatte ist gelenkig mit der Unterschenkelschiene verbunden, wobei sich das Knöchelgelenk auf der Höhe des natürlichen Knöchelgelenkes auf der Höhe des natürlichen Knöchelgelenkes befindet. Über Befestigungsgurte wird die Unterschenkelschiene an dem Patientenunterschenkel angelegt, der Fuß wird auf die Fußschale oder Fußplatte gesetzt und während der Schwungphase über eine Feder unterstützt. Eine solche Anpassung ist aufwändig und erfordert eine große Erfahrung eines Orthopädietechnikers bei der plastischen Verformung der massiven Metallschienen.

Die US 6,540,703 B1 betrifft eine Orthese zur postoperativen Anwendung zur Verhinderung einer Hüftluxation mit einem Beckengurt und einer Oberschenkelmanschette die über ein Gelenk verschwenkbar miteinander verbunden sind. Das Gelenk wird zwischen zwei Metallschienen ausgebildet, die einmal an dem Beckengurt und einmal an der Oberschenkelmanschette gelagert sind. In den jeweiligen Schienen sind Langlöcher angeordnet, entlang derer der Beckengurt beziehungsweise die Oberschenkelmanschette längsverschieblich gelagert sind. An dem Beckengurt und der Oberschenkelmanschette sind halbkreisförmige Aufnahmen mit Querschlitzen angeordnet, um eine rotatorische Einstellung um die Längserstreckung der jeweiligen Schiene zu ermöglichen.

Die EP 1 588 678 A1, die der nächste Stand der Technik darstellt, betrifft eine modulare Hüftorthese mit einem Orthesenteil das aus einem Beckenring, einem Unterschenkteil, einem Condylenteil und Verbindungsmitteln mit einem Gelenk besteht, wobei die Verbindungsmittel den Beckenring mit dem Condylenteil verbinden. Das Oberschenkelteil ist entweder zwischen dem Beckenring und dem Condylenteil mit den Verbindungsmitteln verbunden oder ist ein Bestandteil der Verbindungsmittel. Das Gelenk ermöglicht ein Beugen des Oberschenkels und weist mehrere Gelenkachsen auf, die gemeinsam eine Bewegungsfläche für eine Bewegung des Condylenteils und des Oberschenkelteils relativ zu dem Beckenring definieren.

Die WO 90/00879 A1 betrifft ein System zum Erfassen der relativen Winkelausrichtung zweier beweglicher, benachbarter Skelettsegmente mit ersten und zweiten Gliedern, die miteinander um eine Schwenkachse verlagerbar gekoppelt sind, um sich zusammen mit den Segmenten zu bewegen und einen veränderlichen Winkel zwischen sich zu bilden. Befestigungsmittel sind zum Festlegen der Glieder und Sensoren an den ersten und zweiten Gliedern zum Erfassen des Winkels vorgesehen.

Aufgabe der vorliegenden Erfindung ist es, eine Verstelleinrichtung und einer Orthese bereitzustellen, mit denen eine verbesserte Anpassung orthetischer Komponenten zueinander erfolgen kann, um eine Orthese besser an einem Patienten ausrichten zu können.

Diese Aufgabe wird durch eine Verstelleinrichtung mit den Merkmalen des Hauptanspruches und einer Orthese mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die Verstelleinrichtung zur Verstellung zumindest zweier orthetischer Komponenten zueinander, mit einem ersten Halter, der eine erste Befestigungseinrichtung zur Festlegung einer ersten orthetischen Komponente aufweist, und eine zweiten Halter, der eine zweite Befestigungseinrichtung zur Festlegung an einer zweiten orthetischen Komponente aufweist, sieht vor, dass die beiden Halter in zumindest zwei Orientierungen zueinander verschieblich und miteinander fixierbar gelagert sind. Durch die Ausgestaltung der Verstelleinrichtung aus zwei an den jeweils zueinander auszurichtenden an der fixierbaren orthetischen Komponenten befestigbaren Haltern ist es möglich, eine Verstellbarkeit der orthetischen Komponenten in mehreren Ebenen zueinander zu ermöglichen, wodurch die Gestaltungsfreiheit hinsichtlich der Dimensionierung und der Verstellrichtungen erhöht wird. Mit der Verstelleinrichtung ist es möglich, standardisierte orthetische Komponenten untereinander und aber auch individuell angepasste orthetische Komponenten wie Aufnahmeschalen von Gliedmaßen mit Standardkomponenten sehr variabel zu koppeln, sodass die Funktionsfähigkeit der fertigen Orthese gewährleistet wird. Der Verstellumfang und die Verstellrichtung sind nicht durch die konstruktiven Gegebenheiten der orthetischen Komponenten selbst limitiert, vielmehr können Verschwenkwege oder Verschiebewege sowie die jeweiligen Richtungen durch die Ausgestaltung der Verstelleinrichtung mit zwei Haltern, die an jeweils einer orthetischen Komponente festlegbar und fixierbar sind, erweitert werden. Durch die verschiebliche und aneinander fixierbare Lagerung in der Halter aneinander in zumindest zwei unterschiedlichen Orientierungen können die Komponenten der Orthese einfach, schnell, reversibel und an den jeweiligen Patienten optimiert angepasst werden.

Die Erfindung sieht vor, dass jeder Halter einen Basisträger aufweist, an dem jeweils zumindest ein Kopplungsabschnitt angeordnet oder ausgebildet ist, wobei der jeweilige Kopplungsabschnitt von dem Basisträger abragt. An dem Basisträger kann die jeweilige orthetische Komponente festgelegt sein, über die Kopplungsabschnitte, die von dem jeweiligen Basisträger abragen, kann eine Tiefeneinstellung oder ein Tiefenausgleich bewirkt werden, gegebenenfalls in Kombination mit einer Längsverschieblichkeit, sodass der Abstand der orthetischen Komponenten voneinander den Kopplungsabschnitt einfach variiert werden kann. An dem jeweiligen Basisträger können Befestigungseinrichtungen angeordnet sein, mit denen jeweilige orthetische Komponente festgelegt oder fixiert werden kann. Wenn der Kopplungsabschnitt orthogonal von dem Basisträger abragt, kann bei einer längsverschieblichen Lagerung der Kopplungsabschnitte eine rein orthogonale Verlagerung der Basisträger voneinander weg oder aufeinander zu durchgeführt werden. Bei einer geneigten Orientierung der Kopplungsabschnitte relativ zu dem Basisträger abweichend von einem rechten Winkel, insbesondere in einem stumpfen Winkel, der größer als 90° ist, ist bei der Verschiebung entlang der Kopplungsabschnitte eine überlagerte Bewegung die Folge, bei der nicht nur die Basisträger voneinander weg oder aufeinander zu verlagert werden, sondern auch deren Ausrichtung zueinander verändert und der Abstand der Kopplungsabschnitte angepasst wird.

In einer Weiterbildung der Erfindung ist es vorgesehen, dass jeweils zwei einander gegenüberliegende Kopplungsabschnitte an den Basisträger ausgebildet oder befestigt sind, sodass eine Art Gabel oder eine U-förmige Ausgestaltung entsteht, wodurch eine besonders stabile Zuordnung der beiden Halter zueinander und damit auf der orthetischen Komponenten zueinander ermöglicht wird. Über die beiden von dem Basisträger abragenden Kopplungsabschnitte kann bei einer Festlegung der Kopplungsabschnitte eine gleichmäßig verteilte Kraftleitung über zwei einander gegenüberliegende Kopplungsabschnittspaare erfolgen, was eine stabile Zuordnung der orthetischen Komponenten zueinander ermöglicht.

Bei einer Ausgestaltung mit zwei einander gegenüberliegenden Kopplungsabschnitten an einem Basisträger ist in einer Weiterbildung vorgesehen, dass die Kopplungsabschnitte eines ersten Halters einander zugewandte Innenflächen mit einem lichten Abstand zwischen sich aufweisen, wobei die Kopplungsabschnitte des zweiten Halters einander abgewandte Außenseiten aufweisen. Der Abstand der Außenseiten der zweiten Kopplungsabschnitte zueinander entspricht dabei dem lichten Abstand zwischen den beiden Innenseiten oder Innenflächen der Kopplungsabschnitte des ersten Halters. Dadurch kann der zweite Halter zwischen den beiden Kopplungsabschnitten des ersten Halters positioniert werden, sodass der erste Halter den zweiten Halter von außen umschließt. Hierdurch wird beispielsweise verhindert, dass der zweite Halter sich unkontrolliert von dem ersten Halter entfernen kann, darüber hinaus wird über die Kopplungsabschnitte des ersten Halters eine Führung für den zweiten Halter bereitgestellt. Der lichte Abstand zwischen den Innenflächen muss nur im Wesentlichen dem Außenabstand der Außenseiten der zweiten Kopplungsabschnitte entsprechen.

In einer Weiterbildung der Erfindung ist vorgesehen, dass die beiden Kopplungsabschnitte an einem Basisträger verschieblich zueinander und in ihrem Abstand zueinander fixierbar an den Basisträger gelagert sind, wodurch der Abstand der Kopplungsabschnitte an dem Basisträger zueinander einstellbar ist. Dies erleichtert beispielsweise die Anordnung des zweiten Halters innerhalb des ersten Halters und ermöglicht die Kombination mehrere unterschiedlicher Bauarten oder Dimensionierungen eines Halters mit denen eines anderen Halters. Die beiden verschieblichen Kopplungsabschnitte können in Richtung aufeinander vorgespannt sein, beispielsweise über eine Feder, einen Magneten oder eine entsprechende Kombination aus einem ferromagnetischen Teil und einem Magneten, beispielsweise als Führung des ferromagnetischen Teils. Dadurch haben die beiden Kopplungsabschnitte die Tendenz, sich aufeinander zu zu bewegen, sodass beispielsweise bei einer Ausgestaltung der Verstelleinrichtung mit einen innenliegenden Halter mit zwei Kopplungsabschnitten und einem außenliegenden Halter, bei dem die Kopplungsabschnitte an der Außenseite der Kopplungsabschnitte des ersten Halters anliegen, eine leichte Haltekraft ausgeübt wird, sodass eine Vorfixierung der Halter aneinander erfolgen kann. Grundsätzlich ist es auch möglich, dass die Kopplungsabschnitte in entgegengesetzter Richtung federbelastet und mit einer Kraft beaufschlagt werden, also dass sie voneinander weg gedrückt werden, um von innen gegen zwei Kopplungselemente eines zweiten Halters zu drücken. Dies bedarf allerdings einer Führung und eines Anschlages, um die Bewegung zu begrenzen, falls die außenliegenden Kopplungsabschnitte fehlen.

Der Halter kann als Winkel oder als Gabel ausgebildet sein, wobei die gabelförmige Ausgestaltung bei zwei aneinander gegenüberliegenden Kopplungsabschnitten realisiert wird. Alternativ ist der Halter als Winkel ausgebildet, wobei hier vergrößerte Biegemomente auf den Halter mit dem Basisträger und dem Kopplungsabschnitt auftreten.

In den Kopplungsabschnitten können Schlitze oder Ausnehmungen ausgebildet und/oder an den Kopplungsabschnitten Führungen angeordnet sein, in denen oder an denen der jeweils andere Kopplungsabschnitt verschieblich gelagert ist. In diesen Schlitzen oder Führungen wird der jeweilige Kopplungsabschnitt geführt, wobei die Schlitze oder Führungen geradlinig oder gebogen ausgebildet sein können, um komplexe Bewegungen durch eine Relativverlagerung der Kopplungsabschnitte zueinander zu ermöglichen. Bevorzugt sind die Ausnehmungen, Schlitze und/oder Führungen in Längserstreckung oder Querstreckung der Kopplungsabschnitte orientiert, um einerseits eine Bewegung der Basisträger voneinander weg und andererseits eine Verschiebung relativ zueinander oder auch eine Verdrehung relativ zueinander zu ermöglichen. In einer Variante der Erfindung ist vorgesehen, dass pro Halter die Ausnehmungen, Schlitze und/oder Führungen nur in einer Orientierung ausgerichtet sind, wobei die Ausnehmungen, Schlitze oder Führungen an unterschiedlichen Kopplungsabschnitten unterschiedlich zueinander, bevorzugt senkrecht zueinander orientiert sind, so dass jeder Kopplungsabschnitt oder jedes Kopplungsabschnittspaar nur für eine Verstellrichtung zuständig und vorgesehen ist. Dies erleichtert die Anwendung für den Orthopädiemechaniker oder den Nutzer der Orthese, da eindeutig festgelegt ist, welche Verlagerung durch welche Führung möglich ist, so dass beispielsweise zur Einstellung eines Abstandes einer ersten orthetischen Komponente wie einer Schiene, zu dem Befestigungselement, beispielsweise einer Aufnahmeschale für eine Gliedmaße, nur eine Befestigungseinrichtung oder eine Fixiereinrichtung für den jeweiligen Kopplungsabschnitt gelöst werden muss.

In einer Weiterbildung der Erfindung ist es vorgesehen, dass die Halter zueinander verdrehbar aneinander gelagert sind, um neben rein translatorischen Verstellungen auch eine rotatorische Verstellung der Halter zueinander und damit auch der orthetischen Komponenten zueinander zu ermöglichen. Durch die verdrehbare Lagerung der Halter zueinander, die in der jeweils verdrehten Stellung auch zueinander fixiert werden können, lässt sich ein weiterer Freiheitsgrad der orthetischen Komponenten zueinander einstellen. Bevorzugt weisen die Kopplungsabschnitte zueinander korrespondierend ausgebildete Wölbungen auf, so dass durch die Wölbungen, beispielsweise eine Wölbung auf der Außenseite der innenliegenden Kopplungsabschnitte mit einer korrespondierenden Innenwölbung der Innenseiten der außenliegenden Kopplungsabschnitte eine rotatorische Führung bereitgestellt wird. Dazu können die Kopplungsabschnitte als Teilmantelflächen ausgebildet sein oder an den jeweils aneinander anliegenden oder zueinander orientierten Oberflächen entsprechenden Teilmantelflächenwölbungen aufweisen, so dass die Basisträger zueinander um eine gemeinsame Achse verdrehbar gelagert sind.

Die Befestigungseinrichtungen, über die die orthetischen Komponenten an den jeweiligen Haltern, insbesondere Basisträgern festgelegt werden können, können als Formschlusselemente oder als Kraftschlusselemente ausgebildet sein. Als Formschlusselement kann ein Gewinde, eine Schraube, eine Klipsverbindung, ein Bajonettverschluss oder eine ähnliche Rast- oder Schraubeinrichtung vorgesehen sein, ebenso kann über eine Steckverbindung mit Bolzen eine formschlüssige Befestigung der orthetischen Komponente an dem jeweiligen Halter oder Basisträger erfolgen. Neben der formschlüssigen Festlegung kann die orthetische Komponente an dem Halter kraftschlüssig, insbesondere geklemmt festgelegt werden, beispielsweise über eine Spange oder eine Schraubklemmung. Die Befestigungseinrichtung kann in Ausnehmungen, Schlitzen und/oder Führungen des Basisträgers verschieblich gelagert sein, insbesondere entlang der Längserstreckung des Basisträgers, um eine weitere Verstelldimension zu ermöglichen. Die orthetische Komponente selbst kann innerhalb der jeweiligen Befestigungseinrichtung ebenfalls verstellbar gelagert sein, beispielsweise verschieblich gelagert, um eine stufenlose Verstellung bei einer klemmenden Halterung zu ermöglichen. Über eine formschlüssige Verriegelung und Befestigung der orthetischen Komponente an der Befestigungseinrichtung wird bevorzugt eine Fixierung in diskreten Abständen an vorbestimmten Stellen erreicht.

Eine der Komponenten kann als Orthesenschiene ausgebildet sein, während eine andere Komponente bevorzugt als Halteschale oder Aufnahme oder eine Befestigungseinrichtung für eine Gliedmaße oder den Rumpf ausgebildet sein kann. Die Gliedmaße oder den Rumpf aufnehmende Komponente kann über Befestigungselemente, wie Spanngurte, Klettverschlüsse oder dergleichen an der Gliedmaße fixiert werden. Die Orthesenschiene kann gelenkig mit einer weiteren orthetischen Komponente gekoppelt sein, beispielsweise einer weiteren Orthesenschiene, die ebenfalls über die Verstelleinrichtung und eine Halteschale miteinander gekoppelt werden kann. Beispielsweise kann eine Beinorthese an dem Oberschenkel und dem Unterschenkel über jeweilige Halteschalen festgelegt werden. Die Halteschalen werden an der jeweils für den Nutzer vorteilhaftesten Position befestigt. Über die Verstelleinrichtung wird dann die Kopplung zu einer standardisierten Beinorthese mit einer Oberschenkelschiene, einem Orthesenkniegelenk und einer Unterschenkelschiene hergestellt. Die Positionierung des Orthesengelenkes erfolgt relativ zu dem natürlichen Kniegelenk in einer optimierten Position, was durch die Verstelleinrichtung mit der Vielzahl der Einstellmöglichkeiten sowohl in Längserstreckung der jeweiligen Orthesenschiene als auch hinsichtlich des Abstandes, der Rotation der Aufnahmeschale relativ zu der Längserstreckung der jeweiligen Orthesenschiene sowie der Höheneinstellung sowie der Einstellbarkeit des Abstandes der Orthesenschiene relativ zu dem Oberschenkel und dem Unterschenkel leicht erfolgen kann.

Eine Variante der Erfindung sieht vor, dass an den Kopplungsabschnitten und/oder dem Basisträger Ausrichtmarkierungen oder Skalen angeordnet oder ausgebildet sind, um dem Nutzer oder dem Orthopädietechniker einerseits Hinweise auf eine vorgegebene Verstellung und andererseits eine leichte Auffindbarkeit einer einmal gefundenen Einstellung nach beispielsweise Auswechseln einer Komponente zu ermöglichen. Auch können unterschiedliche Ausrichtungen zu Testzwecken ausprobiert werden, wobei diese Ausrichteinstellungen anhand der Markierungen leicht erkannt und festgehalten werden können, so dass quantitative und qualitative Aussagen über die jeweilige Orientierung der Komponenten zueinander getroffen werden können.

An den Haltern sind Feststeller angeordnet oder ausgebildet, die die jeweiligen Halter zueinander fixieren. Dadurch wird eine lösbare Fixierung der Halter zueinander und damit der orthetischen Komponenten zueinander erreicht. Über die Feststeller können die Kopplungsabschnitte der Halter zueinander fixiert werden, so dass eine Ausrichtung der Orthese zu den Gliedmaßen oder den Rumpf leicht erfolgen kann. Die Feststeller können als Schrauben, Gewinde mit Mutter, Klemmelemente, Klemmknebel und/oder Keile ausgebildet sein, wobei die Feststeller in Führungen, Schlitzen oder Ausnehmungen der Kopplungselemente geführt sein können, um eine kompakte Bauform zu erreichen.

Ausführungsbeispiele der Erfindung werden anhand der beigefügten Figuren näher erläutert. Gleiche Bezugszeichen bezeichnen gleiche Komponenten. Es zeigen:
- Figur 1 -: eine Seitenansicht und eine Draufansicht einer Verstelleinrichtung in einer Grundstellung;
- Figur 2 -: eine Seitenansicht gemäß Figur 1 mit einer längs einer Orthesenschiene verlagerten Verstelleinrichtung;
- Figur 3 -: eine Seitenansicht und eine Draufsicht einer entlang eines Basisträgers verschobenen orthetischen Komponente;
- Figur 4 -: eine Seitenansicht und eine Draufsicht einer tiefenverstellten Verstelleinrichtung gemäß Figur 3;
- Figur 5 -: eine Seitenansicht und eine Draufsicht einer winkelverstellten Verstelleinrichtung;
- Figur 6 -: eine Frontalansicht einer Beinorthese;
- Figur 7 -: eine Seitenansicht einer Orthese gemäß Figur 6;
- Figur 8 -: eine Draufsicht auf eine Orthese gemäß Figur 6;
- Figur 9 -: drei Darstellungen einer ersten Variante einer Verstelleinrichtung;
- Figur 10 -: drei Darstellungen einer Variante der Figur 9 mit verschobener erster Komponente;
- Figur 11 -: eine Variante der Figur 9 mit verschobener Befestigungseinrichtung;
- Figur 12 -: eine Variante der Figur 11 mit verschobenen Haltern; sowie
- Figur 13 -: eine Variante der Figur 12 mit zueinander verdrehten Haltern.

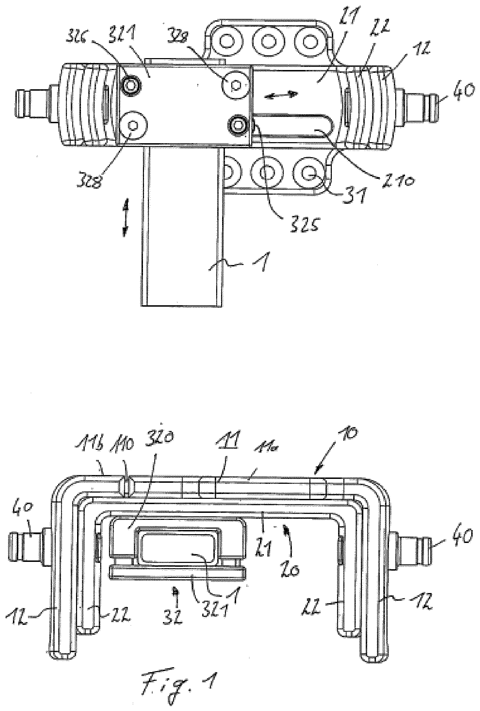

Figur 1 zeigt zwei Ansichten einer Verstelleinrichtung zur verstellbaren Festlegung zwei orthetischer Komponenten, von denen nur eine erste orthetische Komponente 1 in Gestalt einer Orthesenschiene gezeigt ist. Die Verstelleinrichtung weist zwei Halter 10, 20 auf und ist in der Figur 1 in der oberen Darstellung in einer Seitenansicht und in der unteren Darstellung in einer Draufsicht gezeigt. Der erste Halter 10 ist zweiteilig aufgebaut und weist einen Basisträger 11 auf, der im Wesentlichen geradlinig ausgebildet ist und von dem zwei Kopplungsabschnitte 12 orthogonal abragen. Der Basisträger 11 des ersten Halters 10 ist zweiteilig ausgebildet und weist einen ersten Teil 11a auf, in dem Führungen 110, z.B. Stifte, des zweiten Teils 11b aufgenommen sind. Entlang dieser Führungen 110 ist der zweite Teil 11b relativ zu dem ersten Teil 11a längsverschieblich geführt, so dass der Abstand zwischen den beiden Kopplungsabschnitten 12 des ersten Halters 10 eingestellt werden kann. Die beiden Teile 11a, 11b können über nicht dargestellte Magnete, Federn oder ähnliche Einrichtungen aufeinander vorgespannt sein, so dass die beiden Teile 11a, 11b stets in eine Ausgangsstellung zurückbewegt und mit einer Kraft in Richtung auf die Ausgangsstellung belastet werden. Der erste Halter 10 ist in Draufsicht U-förmig ausgebildet und weist an dem Basisträger 11 Befestigungseinrichtungen 31 in Gestalt von Bohrungen für Schraubenaufnahmen auf. Die Befestigungseinrichtungen 31 dienen zur Festlegung einer zweiten orthetischen Komponente an dem ersten Halter 10.

In der unteren Darstellung der Figur 1 ist zu erkennen, dass der zweite Halter 20 im Wesentlichen korrespondierend zu dem ersten Halter 10 aufgebaut ist und einen Basisträger 21 sowie zwei rechtwinklig davon abragende Kopplungsabschnitte 22 aufweist. Der zweiter Träger 20 ist einteilig ausgeführt und innerhalb des ersten Halters 10 zwischen dessen Kopplungsabschnitten 12 angeordnet. Die beiden Basisträger 11, 21 liegen aneinander an, die jeweiligen Kopplungsabschnitte 12, 22 sind in der gleichen Richtung ausgerichtet. Auch der zweite Halter 20 ist U-förmig ausgebildet und ebenfalls aus einem metallischen Werkstoff hergestellt. Die beiden Kopplungsabschnitte 22 ragen rechtwinklig von dem Basisträger 21 ab. Zwischen den Kopplungselementen 22 ist an dem Basisträger 21 eine Befestigungseinrichtung 32 für die Orthesenschiene 1 angeordnet. Die Befestigungseinrichtung 32 ist längsverschieblich entlang der Längserstreckung des Basisträgers 21 innerhalb zweier Ausnehmungen 210, von denen nur eine Ausnehmung 210 zu erkennen ist, gelagert. Die zweite Ausnehmung 210 ist durch die zweite Befestigungseinrichtung 32 verdeckt. Zwei Führungszapfen 325 sind in den jeweiligen schlitzförmigen Führungen 210 gelagert und können über Innensechskantschrauben 326 klemmend in den Führungen 210 fixiert werden.

Die Schiene 1 selbst ist zwischen einem U-förmigen Halter 320 und einer Klemmplatte 321 kraftschlüssig über zwei Schrauben 328 gehalten und kann stufenlos innerhalb der zweiten Befestigungseinrichtung 32 entlang der Längserstreckung der Schiene 1 verschoben werden. Die Verschieblichkeit ist durch den Doppelpfeil in der oberen Darstellung der Figur 1 angedeutet. Durch die klemmende Befestigung kann die Position der Verstelleinrichtung mit den beiden Haltern 10, 20 entlang der Längserstreckung der Schiene 1 stufenlos eingestellt werden.

In der oberen Darstellung der Figur 1 ist zu erkennen, dass die Kopplungsabschnitte 12, 22 eine gewölbte Form aufweisen. Die Kopplungsabschnitte 12, 22 sind wie Teilmantelflächen geformt, so dass die beiden Halter 10, 20 relativ zueinander um eine zentrale Schwenkachse verschwenkt werden können. Dazu sind in dem dargestellten Ausführungsbeispiel innerhalb des zweiten Halters 20 Führungen in Gestalt von Ausnehmungen angeordnet, deren Längserstreckung orthogonal zu der Längserstreckung der Kopplungsabschnitte 22 verläuft. Diese Funktionsweise wird später näher erläutert werden. Innerhalb der Führungen sind Feststeller 40 in Gestalt von Schraubbolzen angeordnet, über die eine klemmende Sicherung der Halter 10, 20 aneinander erfolgen kann.

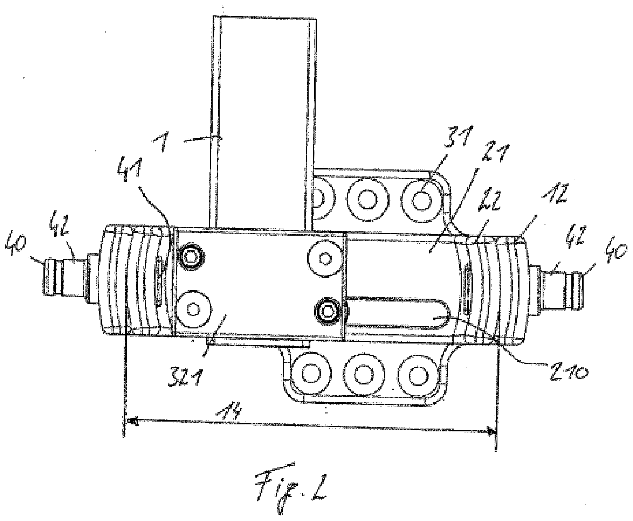

Figur 2 zeigt die Feststelleinrichtung gemäß Figur 1 in einem höhenverstellten Zustand, bei dem die Schiene 1 innerhalb der Befestigungseinrichtung 32 nach oben verschoben worden ist. Die Verstelleinrichtung ist somit relativ zu der ersten orthetischen Komponente 1 nach unten verlagert und dort über die Schrauben 328 klemmend fixiert. In der Figur 2 ist zu erkennen, dass zwischen den äußeren Kopplungsabschnitten 12 ein lichter Abstand 14 ausgebildet ist, innerhalb dessen der zweite Halter 20 mit den Kopplungsabschnitten 22 angeordnet ist. Die gewölbten, äußeren Oberflächen oder Außenseiten 23 der Kopplungsabschnitte 22 des zweiten Halters 20 liegen an den korrespondierend gewölbten inneren Oberflächen oder Innenseiten 13 der Kopplungsabschnitte 12 des ersten Halters an, und zwar so, dass in einem nicht durch einen Feststeller 40 fixierten Zustand die Halter 10, 20 ineinander und aneinander entlang gleiten können.

Die Feststeller 40 weisen einen Bolzen mit einem Kopf 41 und einer Mutter 42 auf, über die die Kopplungsabschnitte 12, 22 der Halter 10, 20 zueinander klemmend fixiert werden können.

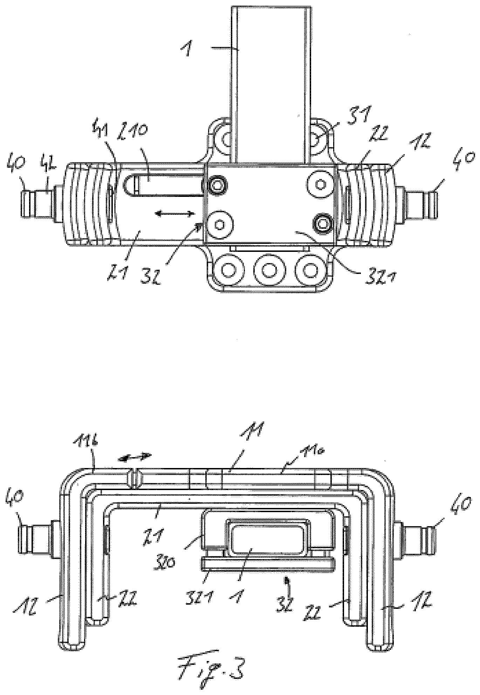

In der Figur 3 ist die Verstelleinrichtung in der oberen Darstellung in einer Seitenansicht und in der unteren Darstellung in einer Draufsicht dargestellt. Die zweite Befestigungseinrichtung 32 ist aus der Position gemäß Figur 2 entlang der Führungen 210 nach rechts verschoben. In der Figur 3 ist zu erkennen, dass eine zweite Führung 210 in dem Basisträger 21 des zweiten Halters 20 geführt ist. Die beiden Führungen 210 sind auf unterschiedlichen Ebenen und versetzt zueinander in dem Basisträger 21 angeordnet, so dass eine Verschiebung über die gesamte Länge des Basisträgers 21 erfolgen kann. Die Befestigungseinrichtung 32 kann somit zwischen den beiden Kopplungsabschnitten 22 des zweiten Halters 20 über den vollständigen inneren Abstand der Kopplungsabschnitte 22 hinweg verschoben werden. Der Doppelpfeil an dem ersten Halter 10 deutet die Verschieblichkeit des zweiten Teils 11b an. Die Feststeller 40 können über die Mutter 42 und den Bolzen mit dem Schraubenkopf 41 an dem zweiten Halter 20 dauerhaft festgelegt werden.

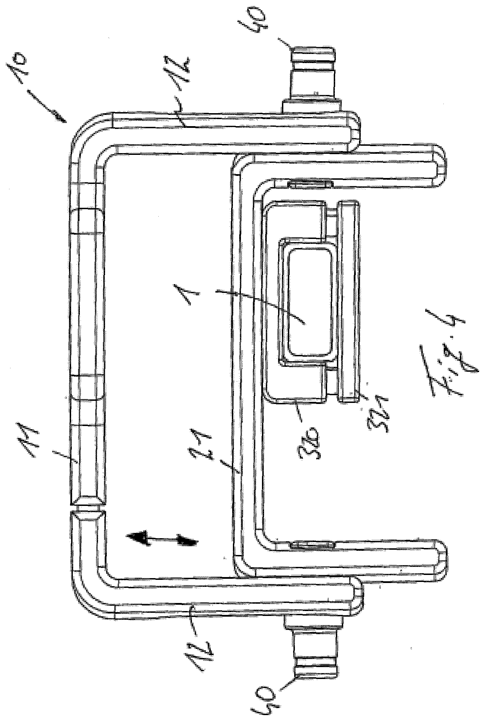

Figur 4 zeigt eine weitere Variante der Erfindung, bei der innerhalb der ersten Kopplungsabschnitte 12 des ersten Halters 10 Längsführungen 121 eingearbeitet sind, so dass der zweite Halter 20 entlang der Längserstreckung der Kopplungsabschnitte 12 verschoben werden kann. Die Verschieblichkeit ist durch den Doppelpfeil angedeutet. Durch die Lagerung des zweiten Halters 20 in den Längsführungen entlang der Kopplungsabschnitte 12 im ersten Halter 10 kann der Abstand zwischen den Basisträgern 11, 21 verändert und eingestellt werden. Durch Anziehen der Feststeller 40 ist es möglich, die jeweils gewünschte Position der Halter 10, 20 und damit auch der beiden orthetischen Komponenten 1, 2 zueinander einzustellen.

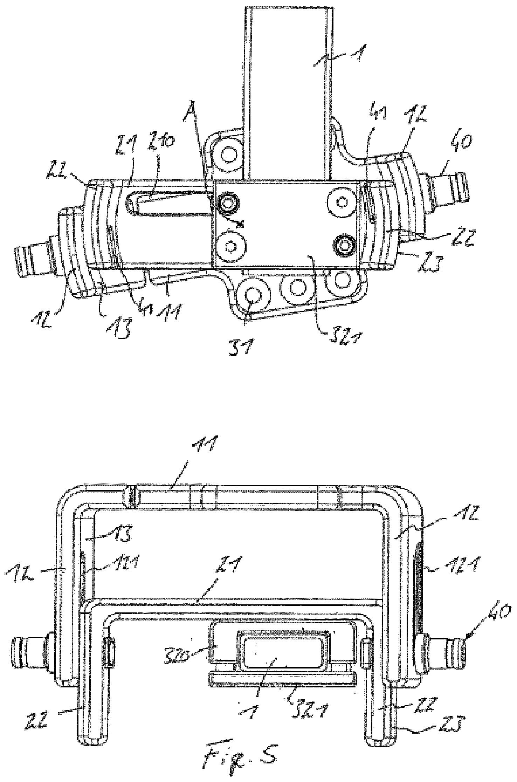

Figur 5 zeigt in der oberen Darstellung in Seitenansicht und in der unteren Darstellung in Draufsicht die Verstelleinrichtung in einer Stellung, in der die Halter 10, 20 zueinander verdreht sind. Innerhalb der Kopplungsabschnitte 22 des zweiten Halters 20 sind senkrecht zu deren Längserstreckung nicht zu erkennende Führungen eingearbeitet, in denen die Schraubenköpfe 41 entlanggleiten. Die Schraubenköpfe 41 sind in Nuten innerhalb der Kopplungsabschnitte 22 geführt, so dass diese nicht über die inneren Oberflächen der Kopplungsabschnitte 22 überstehen. In der oberen Darstellung der Figur 5 ist zu erkennen, dass die beiden Halter 10, 20 zueinander um eine Schwenkachse A zueinander verschwenkt sind. Die mit einem gleichen Radius versehenen, aneinander abgleitenden Oberflächen 23, 13 der jeweiligen Kopplungsabschnitte 22, 12 liegen aneinander an, die Basisträger 11, 21 sind zueinander verkippt, wodurch der erste Halter 10 relativ zu dem zweiten Träger 20 und damit auch zu der fest an dem zweiten Halter 20 montierten Orthesenschiene 1 verschwenkt werden kann. In der unteren Darstellung der Figur 5 sind die Längsführungen 121 innerhalb der ersten Kopplungsabschnitte 12 des ersten Halters 10 zu erkennen, entlang derer die Feststeller 40 entlanggeführt werden können. Die Führungen 121 sind als Langlöcher ausgebildet und weisen umlaufende Vertiefungen auf, innerhalb derer die Muttern 42 entlanggeführt sind.

Die Verstelleinrichtung gemäß der Figuren 1 bis 5 ermöglicht somit eine Verstellung der beiden Halter 10, 20 zueinander entlang der Längserstreckung der jeweiligen Kopplungsabschnitte 11, 21 über die Längsführungen 121 in den ersten Kopplungsabschnitten 12 sowie eine Verdrehung um die Schwenkachse A über die Führungen innerhalb der Kopplungsabschnitte 22 des zweiten Halters 20, die orthogonal zu der Längserstreckung der ersten Führungen 121 und orthogonal zu der Längserstreckung der Kopplungsabschnitte 22 verlaufen. Darüber hinaus ist über die Befestigungseinrichtung 32 eine Verschieblichkeit der ersten Komponente 1 innerhalb der beiden Kopplungsabschnitte 22 des zweiten Halters 20 entlang der Führungen 210 möglich. Zusätzlich kann die Orthesenschiene 1 innerhalb der klemmenden Halterung der Befestigungseinrichtung 32 verschoben werden, so dass insgesamt drei translatorische Freiheitsgrade und ein rotatorischer Freiheitsgrad mit der Verstelleinrichtung abgedeckt sind, so dass vielfältige Einstellmöglichkeiten der Komponenten zueinander, zum Beispiel einer Orthesenschiene und einer Aufnahmeschale, durch die Verstelleinrichtung ermöglicht werden.

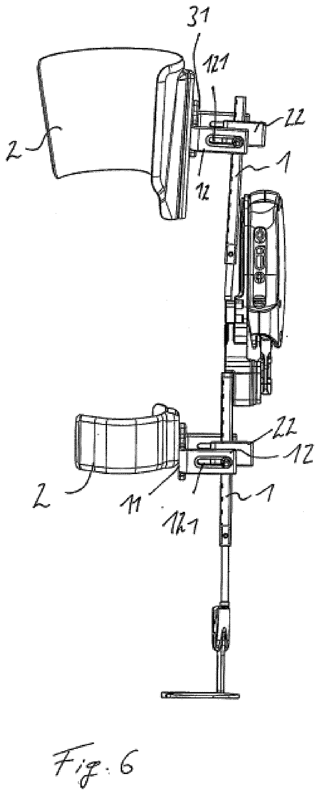

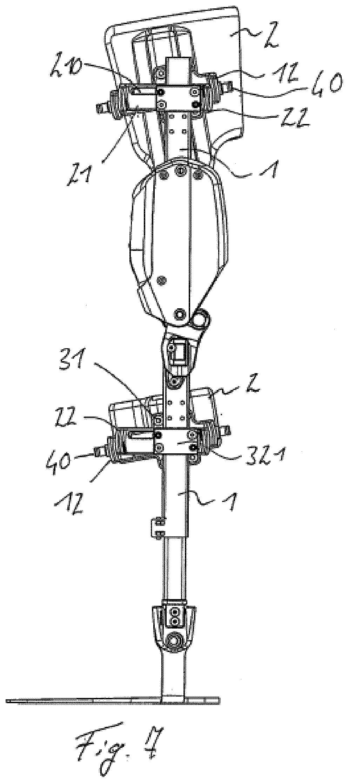

In der Figur 6 ist in einer Frontalansicht eine Beinorthese mit einer Oberschenkelschiene, einer Unterschenkelschiene und einem Fußteil dargestellt. Das Fußteil ist an der Unterschenkelschiene über ein künstliches Knöchelgelenk verschwenkbar gelagert, die Unterschenkelschiene ist über ein Orthesenkniegelenk an der Oberschenkelschiene verschwenkbar gelagert. Die jeweilige Orthesenschiene stellt die erste Orthesenkomponente 1 dar, die bereits in den Figuren 1 bis 5 dargestellt und erläutert wurde. Entlang der Längserstreckung der jeweiligen Orthesenschiene 1 kann die Verstelleinrichtung mit den beiden Haltern 10, 20 durch Lösen der Schrauben der zweiten Befestigungseinrichtung 32 verschoben und anschließend in der gewünschten Höhe wieder stufenlos fixiert werden. Der Frontalansicht sind die beiden Längsführungen 121 in dem ersten Halter 10 zu erkennen, über die eine Beabstandung der jeweils zweiten Komponente 2 zu der Orthesenschiene 1 eingestellt werden kann. Die zweite orthetische Komponente 2 ist im dargestellten Ausführungsbeispiel jeweils eine Aufnahmeschale für eine Gliedmaße, hier eine Oberschenkelaufnahmeschale sowie eine Unterschenkelaufnahmeschale. Über nicht dargestellte Befestigungseinrichtungen wie Gurte oder Schnallen wird die jeweilige Aufnahmeschale 2 an dem Oberschenkel bzw. an dem Unterschenkel festgelegt, so dass eine Kopplung der Gliedmaße mit der funktionellen Komponente der Orthese, nämlich der Führungsschienen 1 mit den Gelenken und gegebenenfalls einer Widerstandseinrichtung, die elektronisch gesteuert ist, hergestellt werden kann. Die jeweils zweite Komponente 2 ist über die erste Befestigungseinrichtung 31 in Gestalt von Schrauben oder Schraublöchern mit dem ersten Halter 10 gekoppelt und festgelegt. Der Figur 6 ist zu entnehmen, dass die Halter 10, 20 zueinander verkippt sind. Die Figur 7 zeigt die Verkippung deutlicher. Der erste Halter 20 ist im Wesentlichen waagerecht orientiert, d.h., dass der Basisträger 10 eine im Wesentlichen waagerecht verlaufende Oberkante aufweist. Hierzu ist die Oberkante des ersten Basisträgers 11 des ersten Halters 10 gegen den Uhrzeigersinn um ca. 20° verkippt, was in ungefähr dem maximalen Kippwinkel um die gemeinsame Achse A entspricht. In der Orthese sind jeweils zwei Verstelleinrichtungen mit jeweils zwei Haltern 10, 20 angeordnet, um die Aufnahmeschalen 2 relativ zu den Orthesenschienen 1 und zueinander auszurichten und eine optimierte Anpassung an dem Patienten zu ermöglichen.

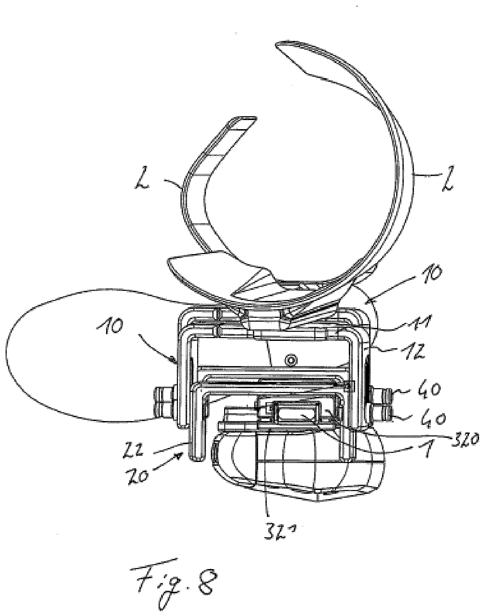

In der Figur 8 ist in einer Draufsicht die Orthese mit der Oberschenkelschiene 1, insgesamt vier Haltern 10, 20 sowie den beiden zweiten orthetischen Komponenten 2 in Gestalt von Aufnahmeschalen dargestellt.

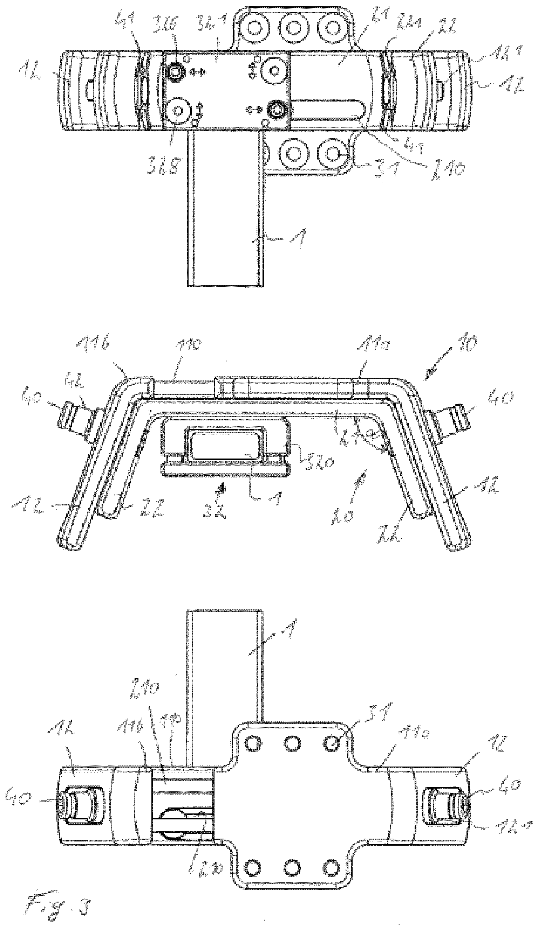

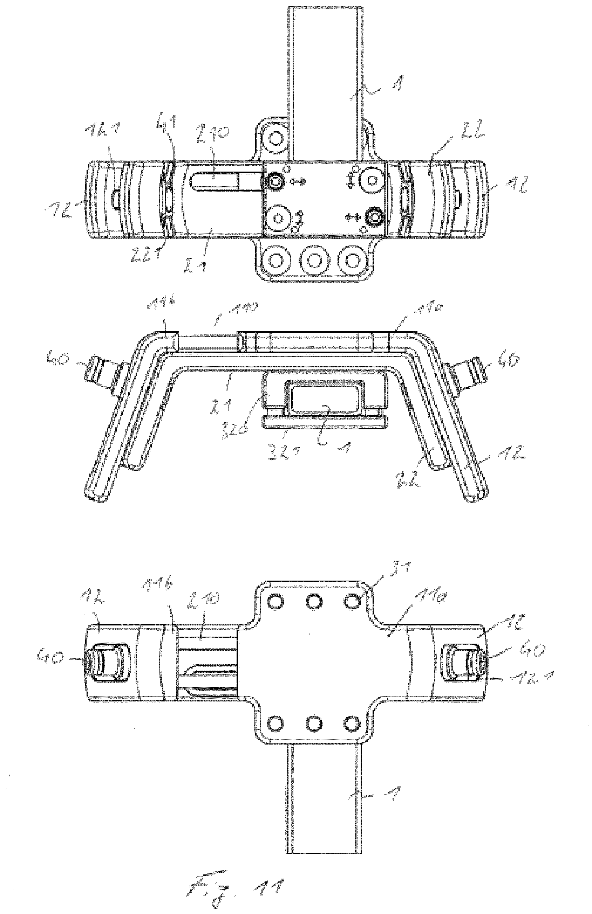

Figur 9 zeigt drei Darstellungen einer Variante der Erfindung in Frontalansicht, Draufsicht und Rückansicht. Gleiche Bezugszeichen bezeichnen gleiche Komponenten. Der grundsätzliche Aufbau der Verstelleinrichtung entspricht derjenigen, die in Figur 1 dargestellt ist. Insofern gelten die Ausführungen hierzu entsprechend. Im Unterschied zu der Ausführungsform gemäß Figur 1 sind die Halter 10, 20 nicht mit rechtwinklig zu einem Basisträger 11, 21 orientierten Kopplungsabschnitten 12, 22 ausgestattet, sondern weisen einen Winkel a zwischen dem Basisträger 11, 21 und den Kopplungsabschnitten 12, 22 auf, der größer als 90° ist und einen stumpfen Winkel ausbildet. Der Winkel a zwischen dem Basisträger 11, 21 und den jeweiligen Kopplungsabschnitten 12, 22 ist bevorzugt in einem Winkelbereich zwischen 91° und 130° ausgebildet. Aufgrund der zweiteiligen Ausgestaltung des ersten Halters 10 ist es möglich, den Basisträger 11 des ersten Halters 10 in seiner Länge zu verändern. In der Figur 9 ist der Basisträger 11 des ersten Halters 10 in seiner maximalen Länge dargestellt, der zweite Halter 20 liegt mit seinem Basisträger 21 an dem Basisträger 11 des ersten Halters 10 an. Die zweite Befestigungseinrichtung 32 befindet sich in der Frontalansicht und in der Draufsicht gesehen ganz links innerhalb den Ausnehmungen 210 in dem zweiten Halter 20. Die erste orthetische Komponente 1 in Gestalt einer Schiene ist in der zweiten Befestigungseinrichtung 32 klemmend gehalten. Die Schrauben 328 verhindern eine Verschieblichkeit der ersten orthetischen Komponente 1 in dessen Längserstreckung, in der oberen Darstellung der Figur 9 nach oben oder nach unten. Dies ist durch die Doppelpfeile an den Schrauben 328 angedeutet. Die Schrauben 326 legen die zweite Befestigungseinrichtung 32 an dem zweiten Halter 20 fest. Die zweite Befestigungseinrichtung 32 ist nach rechts und links verschieblich in den Ausnehmungen 210 gelagert und kann durch Lösen und Anziehen der Schrauben 326 in jeder beliebigen Position zwischen der ganz linken Stellung, wie sie in der Figur 9 dargestellt ist, und einer ganz rechten Darstellung, wie sie in der Figur 11 gezeigt ist, verschoben und klemmend fixiert werden.

Die Entfernung der Basisträger 11, 21 voneinander kann über eine Verlagerung des zweiten Halters 20 relativ zu dem ersten Halter 10 entlang der Ausnehmungen 121 in den beiden Kopplungsabschnitten 12 des ersten Trägers 10 erfolgen. Über die Feststeller 40 mit Kopf 41 und Mutter 42 kann eine lösbare und stufenlose Einstellung entlang der Ausnehmungen 121 erfolgen. In der Darstellung gemäß Figur 9 befindet sich der zweite Halter 20 in einer maximal angenäherten Stellung an den ersten Halter 10.

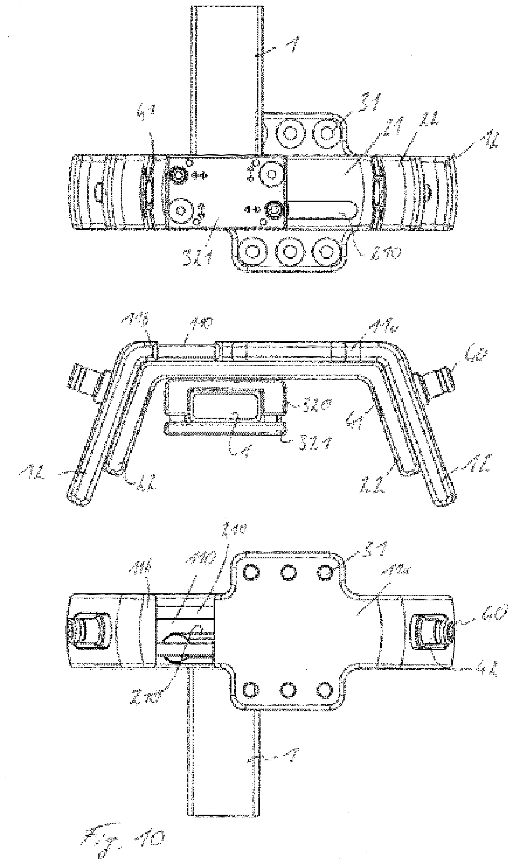

Figur 10 zeigt eine Variante der Figur 9, bei der die erste orthetische Komponente 1 in Gestalt einer Orthesenschiene innerhalb der zweiten Befestigungseinrichtung 32 verschoben ist. Über diese Verschiebung und Fixierung durch die Schrauben 326 ist beispielsweise eine Höheneinstellung der zweiten orthetischen Komponente, die nicht dargestellt ist, relativ zu der ersten orthetischen Komponente 1 möglich.

Figur 11 zeigt eine weitere Variante der Erfindung, bei der die zweite Befestigungseinrichtung 32 ganz nach rechts verschoben ist, gesehen in der Frontalansicht und Draufsicht. Es ist zu erkennen, dass die linke Ausnehmung 210 oberhalb der ersten Ausnehmung 210, die in den Figuren 9 und 10 zu erkennen ist, angeordnet ist.

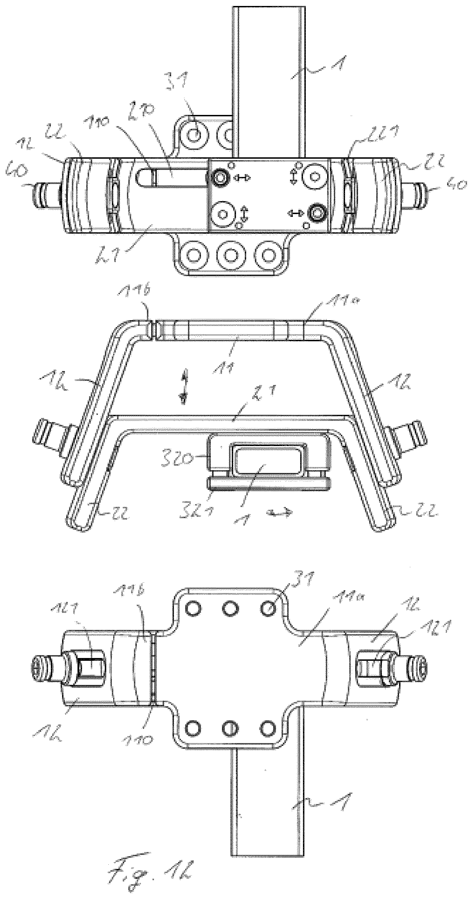

Figur 12 zeigt eine Variante der Figur 11, bei der insbesondere in der Draufsicht, also in der mittleren Darstellung der Figur 12, zu erkennen ist, dass sich der zweite Halter 20 bezüglich der Basisträger 11, 21 in einer maximal entfernten Stellung befindet. Aufgrund der Schrägstellung der Kopplungsabschnitte 12, 22 relativ zu dem jeweiligen Basisträger 11, 21 werden bei einer Längsverschiebung des zweiten Halters 20 entlang der Ausnehmung 121 in den Kopplungsabschnitten 12 des ersten Trägers 10 die beiden Teile 11a, 11b des Halters 10 aufeinander zu bewegt und befinden sich bei einer maximalen Entfernung der Basisträger 11, 21 voneinander in einem maximal angenäherten Zustand und können aneinander anliegen. Die erste Befestigungseinrichtung 31 kann somit in der Tiefe relativ zu der zweiten Befestigungseinrichtung 32 verstellt werden, somit kann die erste orthetische Komponente 1 in einer zweiten Richtung oder Orientierung zu der zweiten orthetischen Komponente 2 verlagert werden. Die Bewegungsrichtungen sind durch die Doppelpfeile in der Figur 12 angedeutet. Die beiden orthetischen Komponenten 1, 2 können in unterschiedlichen, senkrecht zueinander orientierten Richtungen zueinander verschoben oder verlagert werden.

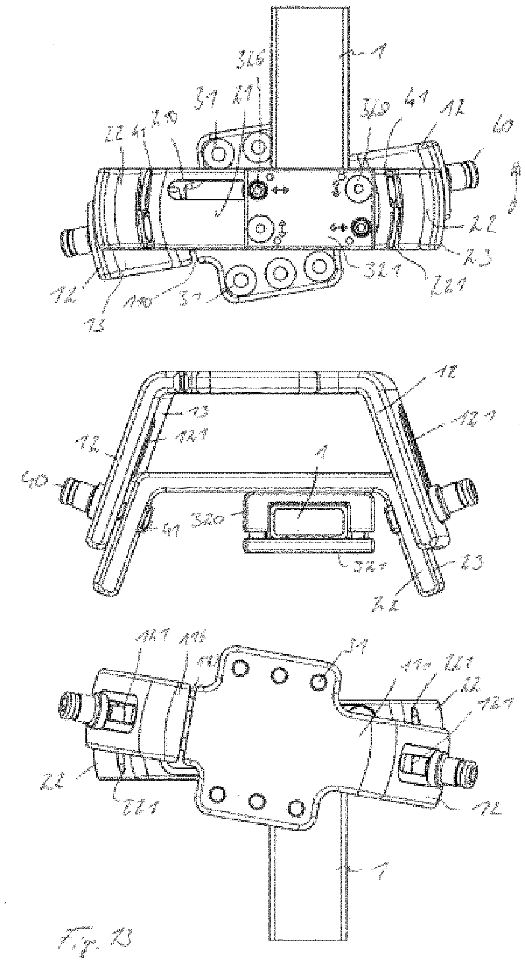

In der Figur 13 ist eine winkelverstellte Verstelleinrichtung ähnlich wie in Figur 5 gezeigt. In dem zweiten Halter 20 sind in den beiden Kopplungsabschnitten 22 Ausnehmungen 221 in Gestalt von Spitzen ausgebildet, die es zusammen mit der gerundeten Ausgestaltung der Außenseite 23 der Kopplungsabschnitte 22 und der gerundeten Innenseite 13 der Kopplungsabschnitte 12 eine Verschwenkbewegung um eine Achse senkrecht auf der Blattebene zu ermöglichen. Die mögliche Verschwenkbewegung ist durch den gebogenen Doppelpfeil in der oberen Darstellung angedeutet. Insgesamt kann sich somit bei einer festen Befestigung der zweiten orthetischen Komponente 2 an der ersten Befestigungseinrichtung 31 Verlagerungen in allen drei translatorischen Freiheitsgraden sowie eine Rotation um einen Freiheitsgrad erreicht werden, so dass die orthetischen Komponenten 1, 2 schnell, einfach und stufenlos zueinander ausgerichtet und an den jeweiligen Patienten angepasst werden können. Alternativ zu einer stufenlosen und klemmenden Verstellung der Halter 10, 20 bzw. der Befestigungseinrichtungen 31, 32 relativ zueinander können auch abgestufte Verlagerungen mit formschlüssigen Verriegelungen durch Rasteinrichtungen ausgebildet sein.

## Patentansprüche

1. Verstelleinrichtung zur verstellbaren Festlegung zumindest zweier orthetischer Komponenten (1, 2) zueinander, mit einem ersten Halter (10), der eine erste Befestigungseinrichtung (31) zur Festlegung einer ersten orthetischen Komponente (1) aufweist, und einem zweiten Halter (20), der eine zweite Befestigungseinrichtung (32) zur Festlegung an einer zweiten orthetischen Komponente (2) aufweist, wobei die Halter (10, 20) in zumindest zwei Orientierungen zueinander verschieblich und aneinander fixierbar gelagert sind, **dadurch gekennzeichnet, dass** jeder Halter (10, 20) einen Basisträger (11, 21) aufweist, an dem jeweils zumindest ein Kopplungsabschnitt (12, 22) angeordnet oder ausgebildet ist, wobei der Kopplungsabschnitt (12, 22) von dem Basisträger (11, 21) abragt.

2. Verstelleinrichtung nach Anspruch 1, **dadurch gekennzeichnet, dass** der Kopplungsabschnitt (12, 22) orthogonal oder in einem stumpfen Winkel (α) von dem Basisträger (11, 21) abragt.

3. Verstelleinrichtung nach Anspruch 1 oder 2, **dadurch gekennzeichnet, dass** jeweils zwei einander gegenüberliegende Kopplungsabschnitte (12, 22) an dem Basisträger (11, 21) ausgebildet oder befestigt sind.

4. Verstelleinrichtung nach Anspruch 3, **dadurch gekennzeichnet, dass** die Kopplungsabschnitte (12) des ersten Halters (10) einander zugewandte Innenflächen (13) mit einem lichten Abstand (14) zwischen sich aufweisen, dass die Kopplungsabschnitte (22) des zweiten Halters (20) einander abgewandte Außenseiten (23) aufweisen und dass der Abstand der Außenseiten (23) der zweiten Kopplungsabschnitte (22) dem lichten Abstand (14) zwischen den Innenflächen (13) entspricht.

5. Verstelleinrichtung nach Anspruch 3 oder 4, **dadurch gekennzeichnet, dass** die Kopplungsabschnitte (12, 22) verschieblich zueinander und/oder in ihrem Abstand zueinander fixierbar an dem Basisträger (11, 21) gelagert sind.

6. Verstelleinrichtung nach Anspruch 5, **dadurch gekennzeichnet, dass** die Kopplungsabschnitte (12, 22) in Richtung aufeinander zu vorgespannt sind.

7. Verstelleinrichtung nach einem der vorstehenden Ansprüche, **dadurch gekennzeichnet, dass** in den Kopplungsabschnitten (12, 22) Schlitze oder Ausnehmungen (121, 221) ausgebildet und/oder an den Kopplungsabschnitten (12, 22) Führungen angeordnet sind, in oder an denen der jeweils andere Kopplungsabschnitt (12, 22) verschieblich gelagert ist.

8. Verstelleinrichtung nach Anspruch 7, **dadurch gekennzeichnet, dass** die Ausnehmungen (121, 221), Schlitze und/oder Führungen in Längserstreckung oder Quererstreckung der Kopplungsabschnitte (12, 22) orientiert sind.

9. Verstelleinrichtung nach Anspruch 8, **dadurch gekennzeichnet, dass** pro Halter (10, 20) die Ausnehmungen (121, 221), Schlitze und/oder Führungen nur in einer Orientierung ausgerichtet sind und die Ausnehmungen (121, 221), Schlitze oder Führungen an unterschiedlichen Kopplungsabschnitten (12, 22) unterschiedlich zueinander orientiert sind.

10. Verstelleinrichtung nach einem der voranstehenden Ansprüche, **dadurch gekennzeichnet, dass** die Halter (10, 20) zueinander verdrehbar aneinander gelagert sind.

11. Verstelleinrichtung nach einem der voranstehenden Ansprüche, **dadurch gekennzeichnet, dass** die Kopplungsabschnitte (12, 22) zueinander korrespondierend ausgebildete Wölbungen aufweisen.

12. Verstelleinrichtung nach Anspruch 11, **dadurch gekennzeichnet, dass** die Kopplungsabschnitte (12, 22) als Teilmantelflächen ausgebildet sind oder Teilmantelflächen aufweisen und die Basisträger (11, 21) um eine gemeinsame Achse (A) verdrehbar gelagert sind.

13. Verstelleinrichtung nach einem der voranstehenden Ansprüche, **dadurch gekennzeichnet, dass** die Befestigungseinrichtung (31, 32) als Formschlusselement oder als Kraftschlusselement ausgebildet ist.

14. Verstelleinrichtung nach einem der voranstehenden Ansprüche, **dadurch gekennzeichnet, dass** die Befestigungseinrichtung (31, 32) in Ausnehmungen (210), Schlitzen und/oder Führungen verschieblich an dem Basisträger (11, 21) gelagert ist.

15. Verstelleinrichtung nach einem der voranstehenden Ansprüche, **dadurch gekennzeichnet, dass** eine der Komponenten (1, 2) als Orthesenschiene ausgebildet ist.

16. Verstelleinrichtung nach einem der voranstehenden Ansprüche, **dadurch gekennzeichnet, dass** eine der Komponenten (1, 2) als Halteschale oder Aufnahme für eine Gliedmaße oder den Rumpf ausgebildet ist.

17. Verstelleinrichtung nach einem der voranstehenden Ansprüche, **dadurch gekennzeichnet, dass** an den Kopplungsabschnitten (12, 22) und/oder dem Basisträger (11, 21) Ausrichtmarkierungen oder Skalen angeordnet oder ausgebildet sind.

18. Verstelleinrichtung nach einem der voranstehenden Ansprüche, **dadurch gekennzeichnet, dass** an den Haltern (10, 20) Feststeller (40) angeordnet sind, die die Halter (10, 20) zueinander fixieren.

19. Verstelleinrichtung nach Anspruch 18, **dadurch gekennzeichnet, dass** die Feststeller (40) als Schrauben (41), Gewinde mit Mutter (42), Klemmelement, Knebel und/oder Keil ausgebildet sind.

20. Verstelleinrichtung nach Anspruch 18 oder 19, **dadurch gekennzeichnet, dass** die Feststeller (40) in den Führungen, Schlitzen oder Ausnehmungen (121, 221) der Kopplungselemente (12, 22) geführt sind.

21. Orthese mit zumindest zwei orthetischen Komponenten (1, 2) und einer Verstelleinrichtung nach zumindest einem der voranstehenden Ansprüche.

## Claims

1. An adjusting device for adjustably securing at least two orthotic components (1, 2) to one another, having a first holder (10) which has a first fastening device (31) for securing a first orthotic component (1), and a second holder (20) which has a second fastening device (32) for securing to a second orthotic component (2), wherein the holders (10, 20) are mounted so as to be able to be mutually displaced and mutually fixed in at least two orientations, **characterized in that** each holder (10, 20) has a main support (11, 21) on which at least one coupling portion (12, 22) is in each case disposed or configured, wherein the coupling portion (12, 22) protrudes from the main support (11, 21).

2. The adjusting device as claimed in claim 1, **characterized in that** the coupling portion (12, 22) protrudes orthogonally or at an obtuse angle (α) from the main support (11, 21).

3. The adjusting device as claimed in claim 1 or 2, **characterized in that** two mutually opposite coupling portions (12, 22) are in each case configured on or fastened to the main support (11, 21).

4. The adjusting device as claimed in claim 3, **characterized in that** the coupling portions (12) of the first holder (10) have mutually facing internal faces (13) having a clearance (14) therebetween, **in that** the coupling portions (22) of the second holder (20) have external sides (23) facing away from one another, and **in that** the spacing of the external sides (23) of the second coupling portions (22) corresponds to the clearance (14) between the internal faces (13).

5. The adjusting device as claimed in claim 3 or 4, **characterized in that** the coupling portions (12, 22) are mounted on the main support (11, 12) so as to be able to be mutually displaced and/or fixed in terms of their mutual spacing.

6. The adjusting device as claimed in claim 5, **characterized in that** the coupling portions (12, 22) are preloaded in the direction toward one another.

7. The adjusting device as claimed in one of the preceding claims, **characterized in that** slots or recesses (121, 221) are configured in the coupling portions (12, 22) and/or guides are disposed on the coupling portions (12, 22), the respective other coupling portion (12, 22) being mounted so as to be displaceable in said slots or recesses or on said guides.

8. The adjusting device as claimed in claim 7, **characterized in that** the recesses (121, 221), slots and/or guides are oriented in the longitudinal extent or transverse extent of the coupling portions (12, 22).

9. The adjusting device as claimed in claim 8, **characterized in that** the recesses (121, 221), slots and/or guides of each holder (10, 20) are aligned in only one orientation, and the recesses (121, 221), slots or guides on different coupling portions (12, 22) are mutually oriented in a different manner.

10. The adjusting device as claimed in one of the preceding claims, **characterized in that** the holders (10, 20) are mounted on one another so as to be mutually rotatable.

11. The adjusting device as claimed in one of the preceding claims, **characterized in that** the coupling portions (12, 22) have curvatures which are configured so as to be mutually corresponding.

12. The adjusting device as claimed in claim 11, **characterized in that** the coupling portions (12, 22) are configured as lateral sub-surfaces, or comprise lateral sub-surfaces, and the main supports (11, 22) are mounted so as to be rotatable about a common axis (A).

13. The adjusting device as claimed in one of the preceding claims, **characterized in that** the fastening device (31, 32) is configured as a form-fit element or as a force-fit element.

14. The adjusting device as claimed in one of the preceding claims, **characterized in that** the fastening device (31, 32) is mounted on the main support (11, 21) so as to be displaceable in recesses (210), slots and/or guides.

15. The adjusting device as claimed in one of the preceding claims, **characterized in that** one of the components (1, 2) is configured as an orthotic brace.

16. The adjusting device as claimed in one of the preceding claims, **characterized in that** one of the components (1, 2) is configured as a support shell or a receptacle for a limb or the torso.

17. The adjusting device as claimed in one of the preceding claims, **characterized in that** alignment markings or graduations are disposed or configured on the coupling portions (12, 22) and/or on the main support (11, 21).

18. The adjusting device as claimed in one of the preceding claims, **characterized in that** locking elements (40) which mutually fix the holders (10, 20) are disposed on the holders (10, 20).

19. The adjusting device as claimed in claim 18, **characterized in that** the locking elements (40) are configured as screws (41), a thread with a nut (42), a clamping element, a toggle, and/or a wedge.

20. The adjusting device as claimed in claim 18 or 19, **characterized in that** the locking elements (40) are guided in the guides, slots or recesses (121, 221) of the coupling elements (12, 22).

21. An orthosis having at least two orthotic components (1, 2) and an adjusting device as claimed in at least one of the preceding claims.

## Revendications

1. Dispositif de réglage pour l'immobilisation réglable d'au moins deux composants orthétiques (1, 2) l'un par rapport à l'autre, comprenant un premier élément de maintien (10) qui présente un premier dispositif de fixation (31) pour l'immobilisation d'un premier composant orthétique (1), et un deuxième élément de maintien (20) qui présente un deuxième dispositif de fixation (32) pour l'immobilisation sur un deuxième composant orthétique (2), les éléments de maintien (10, 20) étant montés de manière à pouvoir être déplacés l'un par rapport à l'autre dans au moins deux orientations et à pouvoir être fixés l'un à l'autre,
**caractérisé en ce que** chaque élément de maintien (10, 20) présente un support de base (11, 21) sur lequel est disposée ou formée au moins une portion d'accouplement (12, 22), la portion d'accouplement (12, 22) faisant saillie du support de base (11, 21).

2. Dispositif de réglage selon la revendication 1,
**caractérisé en ce que** la portion d'accouplement (12, 22) fait saillie orthogonalement ou selon un angle obtus (a) du support de base (11, 21).

3. Dispositif de réglage selon la revendication 1 ou 2,
**caractérisé en ce que** deux portions d'accouplement (12, 22) respectives opposées l'une à l'autre sont formées ou fixées sur le support de base (11, 21).

4. Dispositif de réglage selon la revendication 3,
**caractérisé en ce que** les portions d'accouplement (12) du premier élément de maintien (10) présentent des surfaces intérieures (13) tournées l'une vers l'autre et ayant une distance libre (14) entre elles, **en ce que** les portions d'accouplement (22) du second élément de maintien (20) ont des faces extérieurs (23) détournées l'une de l'autre, et **en ce que** la distance entre les faces extérieures (23) des secondes portions d'accouplement (22) correspond à la distance libre (14) entre les surfaces intérieures (13).

5. Dispositif de réglage selon la revendication 3 ou 4,
**caractérisé en ce que** les portions d'accouplement (12, 22) sont montées sur le support de base (11, 21) de manière à pouvoir être déplacées l'une par rapport à l'autre et/ou à pouvoir être fixées à leur distance l'une par rapport à l'autre.

6. Dispositif de réglage selon la revendication 5,
**caractérisé en ce que** les portions d'accouplement (12, 22) sont précontraintes en direction l'une vers l'autre.

7. Dispositif de réglage selon l'une des revendications précédentes,
**caractérisé en ce que** des fentes ou des évidements (121, 221) sont ménagés dans les portions d'accouplement (12, 22) et/ou des guides sont disposés sur les portions d'accouplement (12, 22), dans ou sur lesquels l'autre portion d'accouplement respective (12, 22) est montée de façon déplaçable.

8. Dispositif de réglage selon la revendication 7,
**caractérisé en ce que** les évidements (121, 221), les fentes et/ou les guides sont orientés dans l'extension longitudinale ou dans l'extension transversale des portions d'accouplement (12, 22).

9. Dispositif de réglage selon la revendication 8,
**caractérisé en ce que**, pour chaque élément de maintien (10, 20), les évidements (121, 221), les fentes et/ou les guides sont orientés dans une seule orientation, et les évidements (121, 221), les fentes ou les guides de différentes portions d'accouplement (12, 22) sont orientés différemment les uns par rapport aux autres.

10. Dispositif de réglage selon l'une des revendications précédentes,
**caractérisé en ce que** les éléments de maintien (10, 20) sont montés l'un sur l'autre de manière à pouvoir tourner l'un par rapport à l'autre.

11. Dispositif de réglage selon l'une des revendications précédentes,
**caractérisé en ce que** les portions d'accouplement (12, 22) présentent des bombements formés de manière à se correspondre.

12. Dispositif de réglage selon la revendication 11,
**caractérisé en ce que** les portions d'accouplement (12, 22) sont réalisées sous forme de surfaces enveloppes partielles ou présentent des surfaces enveloppes partielles, et les supports de base (11, 21) sont montés de manière à pouvoir tourner autour d'un axe commun (A).

13. Dispositif de réglage selon l'une des revendications précédentes,
**caractérisé en ce que** le dispositif de fixation (31, 32) est réalisé sous forme d'élément en coopération de forme ou d'élément en coopération de force.

14. Dispositif de réglage selon l'une des revendications précédentes,
**caractérisé en ce que** le dispositif de fixation (31, 32) est monté sur le support de base (11, 21) de façon déplaçable dans des évidements (210), des fentes et/ou des guides.

15. Dispositif de réglage selon l'une des revendications précédentes,
**caractérisé en ce que** l'un des composants (1, 2) est réalisé sous forme d'attelle orthopédique.

16. Dispositif de réglage selon l'une des revendications précédentes,
**caractérisé en ce que** l'un des composants (1, 2) est réalisé sous forme de coque de maintien ou de logement pour un membre ou pour le tronc.

17. Dispositif de réglage selon l'une des revendications précédentes,
**caractérisé en ce que** des repères ou des échelles d'orientation sont disposés ou formés sur les portions d'accouplement (12, 22) et/ou le support de base (11, 21).

18. Dispositif de réglage selon l'une des revendications précédentes, **caractérisé en ce que** des organes d'arrêt (40) sont disposés sur les éléments de maintien (10, 20) et fixent les éléments de maintien (10, 20) l'un par rapport à l'autre.

19. Dispositif de réglage selon la revendication 18,
**caractérisé en ce que** les organes d'arrêt (40) sont réalisés sous forme de vis (41), de filetages avec écrous (42), d'éléments de serrage, de tourniquets et/ou de cales.

20. Dispositif de réglage selon la revendication 18 ou 19,
**caractérisé en ce que** les organes d'arrêt (40) sont guidés dans les guides, fentes ou évidements (121, 221) des éléments d'accouplement (12, 22).

21. Orthèse comportant au moins deux composants orthétiques (1, 2) et un dispositif de réglage selon l'une au moins des revendications précédentes.
